# FASCICULE DE BREVET EUROPEEN

(11) **EP 3 558 134 B1**
(45) Date de publication et mention de la délivrance du brevet: **14.10.2020**
(21) Numéro de dépôt: 17821674.3
(22) Date de dépôt: 30.11.2017
(51) Int. Cl.: A61B 8/06, A61B 8/00, A61B 8/08, B63C 11/02

(54) **DISPOSITIF DE MESURE DU FLUX SANGUIN**
VORRICHTUNG ZUR MESSUNG DES BLUTFLUSSES
DEVICE FOR MEASURING BLOOD FLOW

(30) Priorité: 23.12.2016 FR 1663309
(43) Date de publication de la demande: 30.10.2019
(73) Titulaire: Azoth Systems, 83190 Ollioules (FR)
(72) Inventeur: QUINSAC, Céline, 75019 Paris (FR); BARBAUD, Axel, 83000 Toulon (FR)
(74) Mandataire: Plasseraud IP
(86) Numéro de dépôt international: PCT/FR2017/053334
(87) Numéro de publication internationale: WO 2018/115617

(56) Documents cités:
- WO-A1-2015/145011
- EDWARD O BELCHER: "Quantification of Bubbles Formed in Animals and Man During Decompression", IEEE TRANSACTIONS ON BIOMEDICAL ENGINEERING, IEEE SERVICE CENTER, PISCATAWAY, NJ, USA, vol. BME-19, no. 6, 2 juin 1980 (1980-06-02), pages 330-338, XP011174588, ISSN: 0018-9294
- CHRISTIAN R GUTVIK ET AL: "Optimal Decompression of Divers [Applications of Control]", IEEE CONTROL SYSTEMS, IEEE, USA, vol. 31, no. 1, 2 février 2011 (2011-02-02), pages 19-28, XP011372726, ISSN: 1066-033X, DOI: 10.1109/MCS.2010.939141

## Description

La présente invention est relative aux dispositifs de mesure du flux sanguin, notamment pour des opérations de détection de bulles de décompression ou autres.

Une telle mesure de flux sanguin permet en particulier aux plongeurs professionnels ou amateurs de contrôler et surveiller leur exposition aux effets de changements de pression, notamment pour prévenir les accidents de décompression.

Le domaine d'application de la présente invention s'étend de manière plus générale à tout type de suivi d'exposition humaine en environnement hyperbare/hypobare, notamment dans le cas des tunneliers, lors de l'utilisation de caissons hyperbares, ou de systèmes de plongée à saturation, ou encore dans le domaine aérospatial, cette liste étant bien évidemment non limitative.

WO2015/145011 propose un procédé de détection acoustiques de bulles dans le sang.

### ARRIERE-PLAN DE L'INVENTION

La présente invention concerne les dispositifs de mesure de circulation sanguine et de détection, par ondes acoustiques, de bulles dans le sang d'une portion du système cardiovasculaire d'une personne.

Pour réaliser les mesures de bulles dans la circulation sanguine d'un individu, on utilise de façon connue un appareil de détection acoustique par effet doppler, notamment une sonde ultrasonore.

Un opérateur formé à la réalisation de mesures Doppler place la sonde ultrasonore sur la poitrine du plongeur et vise une cavité cardiaque d'intérêt, par exemple les cavités cardiaques droites qui correspondent au retour veineux.

L'opérateur guide usuellement le positionnement de la sonde au moyen du signal audio délivré par la sonde, en cherchant à reconnaître le signal doppler associé au flux sanguin de la ou des cavités cardiaques visées et, le cas échéant, les signaux doppler caractéristiques de la présence de bulles.

Lorsque l'opérateur a identifié, à l'oreille, un signal qu'il associe à un positionnement correct de la sonde en face des cavités cardiaques, il procède usuellement à la cotation du signal entendu.

Par le terme « cotation », on entend notamment compter ou estimer le nombre de signaux caractéristiques de bulles entendus parmi les signaux de flux cardiaque.

L'utilisation d'une telle sonde présente plusieurs inconvénients.

Ainsi, l'opérateur doit être formé et exercé à la reconnaissance des signaux audio des cavités visées afin de pouvoir guider « à l'oreille » le positionnement de la sonde pour être capable de reconnaître le signal caractéristique d'un bon positionnement de la sonde. L'opérateur peut ainsi se tromper si son oreille n'est pas suffisamment exercée et la sonde peut se trouver mal positionnée et détecter du signal à un mauvais endroit.

Ceci empêche en pratique l'utilisation d'un tel dispositif et procédé de mesure par un opérateur non formé à la mesure des signaux Doppler.

Par ailleurs, même lorsque l'opérateur est formé à la mise en œuvre d'un tel procédé de positionnement, ce dernier exige d'écouter un signal audio, opération qui n'est pas commode dans un environnement bruité.

Le simple fait de devoir écouter les signaux des bulles de décompression pour positionner la sonde peut également causer un risque non négligeable. En effet, le passage de ces bulles peut être particulièrement sonore et il a été observé qu'il est à même de provoquer une inquiétude ou un stress du plongeur qui les écoute. L'importance de ce dernier inconvénient ne doit pas être sous-estimée car le stress est un facteur qui favorise la survenue d'un accident de décompression.

Il existe donc un besoin pour un dispositif qui permette à un plongeur, ou un opérateur non spécialement formé d'effectuer, des mesures de flux sanguin, notamment pour la détection de bulles de décompression, de manière simple, fiable, reproductible, peu stressante. Il existe également un besoin pour un tel dispositif qui soit peu coûteux et robuste de sorte notamment à pouvoir être utilisé à chaque sortie de plongée et donc dans un environnement extérieur présentant des conditions parfois difficiles (humidité, température, mouvements ...).

### OBJETS DE L'INVENTION

A cet effet, l'invention a pour premier objet un dispositif de mesure du flux sanguin dans une portion de système cardiovasculaire d'un individu, notamment pour des opérations de détection de bulles, le dispositif comprenant
un émetteur acoustique comportant au moins un transducteur acoustique adapté pour émettre un faisceau d'ultrasons et capter un signal ultrasonore réfléchi, et
un boitier dans lequel est accueilli l'émetteur acoustique.

Le dispositif est tel que le boitier comprend une face d'émission, sensiblement plane ou convexe, venant coiffer l'émetteur acoustique pour le protéger d'un environnement extérieur et assurer la transmission du faisceau d'ultrasons et du signal ultrasonore réfléchi,
la face d'émission s'étendant sensiblement dans un plan d'extension verticale comprenant une direction transversale et une direction verticale perpendiculaires de sorte à être apte à venir en contact avec la peau d'un individu selon une direction longitudinale sensiblement perpendiculaire au plan d'extension verticale,
la face d'émission étant terminée à une extrémité selon la direction verticale par une portion de contact de clavicule sensiblement perpendiculaire à la face d'émission et adaptée pour venir en appui contre un os de clavicule de l'individu,
la face d'émission étant terminée à une extrémité selon la direction transversale par une portion de contact d'épaule sensiblement perpendiculaire à la face d'émission et adaptée pour venir en appui contre un os de l'épaule de l'individu,
une distance verticale entre un milieu de zone active de la face d'émission et la portion de contact de clavicule étant inférieure à 20 millimètres, et
une distance transversale entre un milieu de zone active de la face d'émission et la portion de contact d'épaule étant comprise entre 20 et 50 millimètres.

Les bulles susmentionnées peuvent être des bulles de décompression ou toutes autres bulles gazeuses, notamment des bulles gazeuses dues à des embolies.

Dans des modes de réalisation préférés de l'invention, on peut éventuellement avoir recours en outre à l'une et/ou à l'autre des dispositions suivantes :
- la distance transversale entre le milieu de zone active et la portion de contact d'épaule est comprise entre 30 et 40 millimètres;
- la face d'émission est terminée à deux extrémités transversales opposées selon la direction transversale, par deux portions de contact d'épaule sensiblement perpendiculaires à la face d'émission et adaptées pour venir en appui contre un os de l'épaule de l'individu,
   et le milieu de zone active de la face d'émission est situé sensiblement à équidistance des portions de contact d'épaule selon la direction transversale;
- le boîtier comprend une face de calage s'étendant sensiblement dans le plan d'extension verticale et disposée en retrait par rapport à la face d'émission selon la direction longitudinale de sorte à être adaptée pour venir en appui plan contre la clavicule de l'individu, en particulier disposée avec une distance de retrait selon la direction longitudinale comprise entre 5 et 15 millimètres;
- la portion de contact de clavicule relie la face d'émission à la face de calage, en particulier la portion de contact de clavicule est relié à la face de calage en formant un congé de sorte à suivre la courbe d'une clavicule de l'individu;
- le dispositif acoustique comprend en outre au moins un capteur de contact de peau adapté pour détecter un contact de la face d'émission avec la peau de l'individu, en particulier le dispositif comporte deux capteurs de contact disposés de part et d'autre de l'émetteur acoustique selon la direction transversale;
- un transducteur de l'émetteur acoustique est incliné par rapport au plan d'extension verticale de la face d'émission de sorte à ce que la direction d'émission du transducteur soit inclinée en direction de la portion de contact de clavicule;
- l'émetteur acoustique présente un encombrement maximal dans le plan d'extension vertical compris entre 30 et 40 millimètres, en particulier l'émetteur acoustique comporte deux transducteurs acoustiques, plus particulièrement les deux transducteurs acoustiques présentent un angle compris entre 140 et 180° entre leurs plans d'extension respectifs;
- le boîtier accueille en outre une électronique de contrôle et de commande de l'émetteur acoustique, ladite électronique étant disposée sur au moins une carte électronique s'étendant sensiblement selon le plan d'extension verticale, notamment le boîtier présente en outre un encombrement total selon la direction longitudinale inférieure à 50 millimètres;
- l'électronique de contrôle et de commande de l'émetteur acoustique comporte des moyens de communication sans fil avec une station de base telle qu'un smartphone ou un ordinateur.

### BREVE DESCRIPTION DES DESSINS

D'autres caractéristiques et avantages de l'invention apparaîtront au cours de la description suivante de plusieurs de ses formes de réalisation, données à titre d'exemples non limitatifs, en regard des dessins joints.

Sur les dessins :
- la figure 1 est une vue schématique en perspective d'un dispositif selon un mode de réalisation de l'invention,
- la figure 2 est une vue schématique d'un squelette d'un individu illustrant notamment le positionnement d'un dispositif selon l'invention,
- la figure 3 est une vue en coupe du dispositif de la figure 1,
- les figures 4A et 4B sont respectivement des vues de face et de profil du dispositif de la figure 1, et
- les figures 5A, 5B et 5C sont respectivement des vues de derrière, de gauche et de dessus d'un émetteur acoustique d'un dispositif selon l'invention, illustrant la lentille et les transducteurs acoustiques, et
- les figures 6A, 6B et 6C sont des vues de derrière d'un émetteur acoustique d'un dispositif selon l'invention selon trois variantes de réalisation, illustrant en particulier la position et la forme des transducteurs acoustiques.

### DESCRIPTION PLUS DETAILLEE

Sur les différentes figures, les mêmes références désignent des éléments identiques ou similaires.

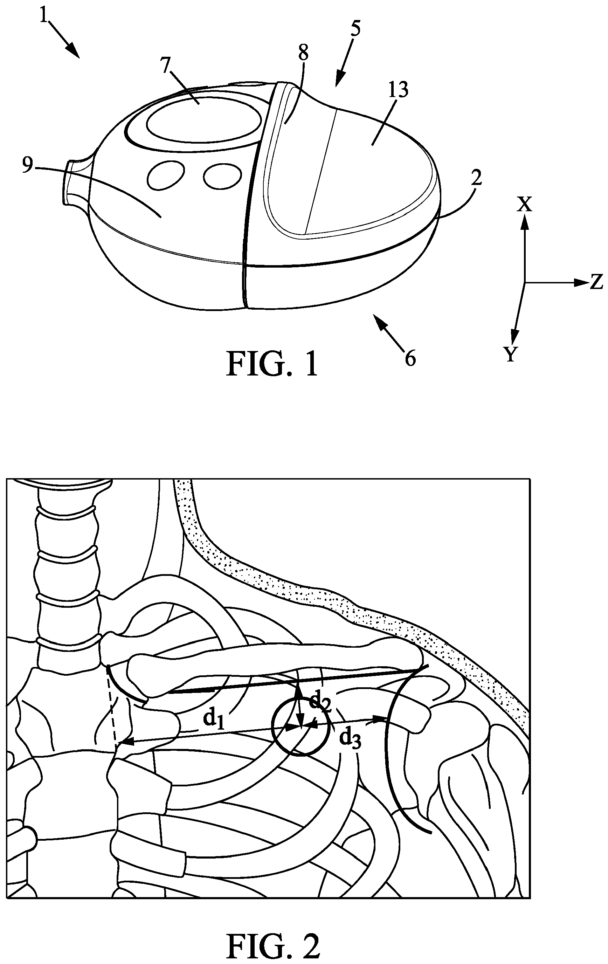

La figure 1 illustre un dispositif 1 de mesure du flux sanguin selon un mode de réalisation de l'invention.

L'une des particularités de ce dispositif est d'être plus spécialement adapté à la mesure du flux sanguin dans une portion de système cardiovasculaire précisé qui est une veine sous-clavière telle qu'illustrée sur la figure 2.

En effet, il a été observé par les inventeurs que les veines sous-clavières, présentent derrière chacune des clavicules d'un individu, permettent une mesure plus reproductible que la mesure sur les cavités cardiaques et en particulier permettent d'obtenir un dispositif offrant un positionnement simplifié comme cela va être décrit dans la suite de la présente description.

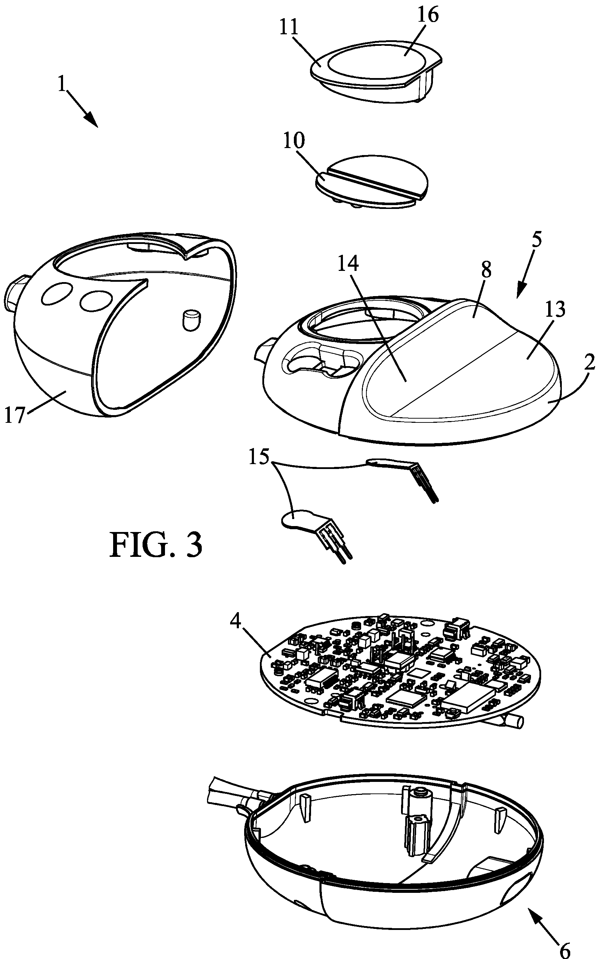

Le dispositif 1 est illustré notamment sur la figure 3 et comporte un boîtier 2 accueillant un émetteur acoustique 3 et une électronique 4 de contrôle et de commande de l'émetteur acoustique.

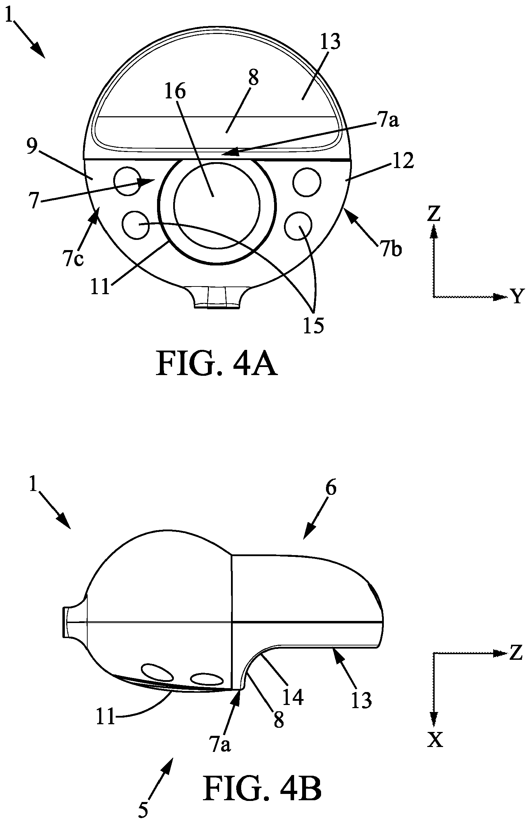

Le boîtier 2 est illustré notamment sur les figures 4A et 4B.

Le boîtier 2 s'étend principalement selon un plan d'extension verticale Y, Z comprenant une direction transversale Y et une direction verticale Z perpendiculaires et accessoirement selon une direction longitudinale X perpendiculaire au plan d'extension verticale.

Dans l'exemple illustré sur les figures, le boîtier 2 présente la forme d'un disque.

Le boîtier 2 comporte ainsi un premier côté 5 et un deuxième côté 6, opposés selon la direction longitudinale X.

Le premier côté 5 est adapté pour venir en contact avec la peau du thorax de l'individu sur lequel est effectuée la mesure de flux sanguin.

Le deuxième côté 6 est adapté pour permettre la prise en main du dispositif 1.

Plus précisément, le premier côté 5 comporte une face d'émission 7 venant coiffer l'émetteur acoustique 3.

Une portion du boîtier peut être entourée d'une housse 17 de contact, notamment une portion de la face d'émission 7. La housse 17 est par exemple réalisée en un matériau élastomère pour limiter les glissements sur la peau.

La face d'émission 7 permet de protéger l'émetteur acoustique 3 de l'environnement extérieur et d'assurer une adaptation d'impédance acoustique avec la peau de l'individu pour la transmission des ultrasons.

La face d'émission peut être formée par un seul constituant ou peut comporter plusieurs sous-éléments. La face d'émission 7 peut notamment comporter une lentille 16 convexe, comme illustré sur la figure 3.

La lentille 16 peut être réalisée en un polyuréthane choisi pour ses propriétés de faible atténuation acoustique.

La face d'émission 7 est sensiblement plane ou légèrement convexe et s'étend sensiblement selon le plan d'extension verticale Y, Z. La face d'émission 7 est terminée à des extrémités verticales et transversales par des portions de positionnement de la sonde particulières qui vont maintenant être décrites.

A une première extrémité 7a selon la direction verticale Z, la face d'émission 7 est terminée par une portion de contact de clavicule 8 sensiblement perpendiculaire à la face d'émission et adaptée pour venir en appui contre un os de clavicule de l'individu selon la direction verticale Z.

Par « venir en appui contre un os de clavicule de l'individu selon la direction verticale », on entend que l'individu peut placer le dispositif contre sa peau en dessous de la clavicule, par exemple de telle sorte que la face d'émission soit placée à plat contre le muscle grand pectoral, puis déplacer le dispositif selon la direction verticale jusqu'à ce que la portion de contact de clavicule 8 vienne en appui contre la clavicule de l'individu par le bas selon la direction verticale.

A une deuxième extrémité 7b selon la direction transversale Y, la face d'émission 7 est terminée par une portion de contact d'épaule 9 sensiblement perpendiculaire à la face d'émission 7 et adaptée pour venir en appui contre un os de l'épaule de l'individu selon la direction transversale Y.

Par « venir en appui contre un os de l'épaule de l'individu selon la direction transversale », on entend que l'individu peut placer le dispositif contre sa peau sur le torse et à proximité de l'épaule, par exemple de telle sorte que la face d'émission soit placée à plat contre le muscle grand pectoral, puis déplacer le dispositif selon la direction transversale jusqu'à ce que la portion de contact d'épaule 9 vienne en appui contre un os de l'épaule de l'individu par le côté selon la direction transversale.

Ledit os de l'épaule de l'individu peut en particulier être une portion de l'omoplate, par exemple une face du processus coracoïde.

Il est important de garantir dans la mesure du possible un contact intime entre la face d'émission 7 et la peau de l'individu. Pour cela, le dispositif peut comporter en outre au moins un capteur de contact 15. Le capteur de contact 15 peut être adapté pour détecter un contact de la face d'émission 7 avec la peau de l'individu.

Le capteur de contact 15 est par exemple un capteur de pression.

Pour confirmer le bon positionnement du dispositif, celui-ci peut en particulier comprendre deux capteurs de contact 15 disposés de part et d'autre de l'émetteur acoustique 3 selon la direction transversale Y comme illustré sur la figure 4A.

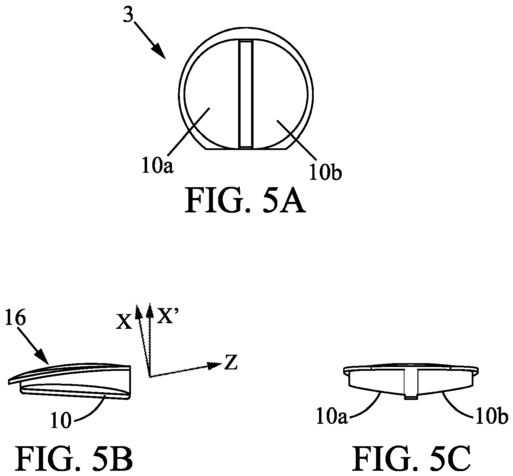

L'émetteur acoustique 3 comporte un ou plusieurs transducteurs acoustiques 10 qui forme une zone active 11 et est illustré plus en détail sur les figures 5A à 5C.

Les transducteurs acoustiques 10 sont par exemple adaptés pour fonctionner dans une gamme comprise entre 2 MHz et 8 MHz, par exemple proche de 2 MHz.

Le signal émis et acquis par les transducteurs acoustique est par exemple défini et traité comme cela est connu par ailleurs dans le domaine de l'échographie vasculaire, par exemple de l'échographie doppler vasculaire.

Sur la figure 5A par exemple l'émetteur acoustique 3 comporte deux transducteurs acoustiques 10 présentant chacun une forme de demi-disque.

Les transducteurs acoustiques 10 de l'émetteur acoustique utilisés lors de la mesure du flux sanguin de l'individu forment une zone active 11 sur la face d'émission 7. Dans l'exemple de la figure 5A, la zone active 11 est ainsi notamment en forme de disque.

Plus précisément, la zone active 11 peut comporter une lentille 16 légèrement convexe, comme illustrée sur la figure 5B, qui permet de réduire la présence de bulle d'air entre les transducteurs acoustiques 10 et la peau de l'individu.

L'émetteur acoustique 3, et en particulier sa zone active 11 peuvent plus précisément présenter un encombrement maximal dans le plan d'extension vertical Y, Z compris entre 30 et 40 millimètres.

La portion de contact de clavicule 8 et la portion de contact d'épaule 9 sont placées par rapport à la zone active 11 et en particulier au milieu de la zone active d'une manière précise qui est illustrée sur la figure 2.

Par « milieu de la zone active », on entend un barycentre géométrique de la zone active.

Ainsi, une distance verticale entre le milieu de zone active 11 et la portion de contact de clavicule 8 est inférieure à 20 millimètres, de préférence inférieure à 15 millimètres.

Par une « distance verticale », on entend une distance mesurée selon la direction verticale Z.

Cette distance verticale est illustrée par la référence d2 sur la figure 2.

Par ailleurs, une distance transversale entre le milieu de zone active 11 et la portion de contact d'épaule 9 est compris entre 20 et 50 millimètres, de préférence entre 30 et 40 millimètres.

Par une « distance transversale», on entend une distance mesurée selon la direction transversale Y.

Cette distance transversale est illustrée par la référence d3 sur la figure 2.

Une autre distance transversale est illustrée par la référence d1 sur la figure 2 et mesure une distance selon l'axe transversal entre le dispositif 1 et la colonne vertébrale d'un individu.

De cette manière, les signaux ultrasonores émis par l'émetteur acoustique 3 sont adaptés pour insonifier une portion de la veine sous-clavière.

Plus précisément, l'émetteur acoustique 3 peut être arrangé pour améliorer encore ladite insonification. Ainsi par exemple au moins un transducteur 10 de l'émetteur acoustique 3 peut être incliné par rapport au plan d'extension verticale Y, Z de la face d'émission 7. En particulier, la direction d'émission X' du transducteur 10 qui est sensiblement orientées selon la direction longitudinale X du dispositif 1 peut être inclinée en direction de la portion de contact de clavicule 8.

Par exemple, un angle entre la direction d'émission X' du transducteur 10 et la direction longitudinale X du dispositif 1 peut être d'environ 5 °.

Les transducteurs acoustiques 10 peuvent ne pas être coplanaires et présenter un angle respectif de sorte à contrôler la largeur du faisceau acoustique à l'endroit de la veine sous-clavière et garantir une meilleure reproductibilité des mesures.

Ainsi dans l'exemple de la figure 5C, deux transducteurs acoustiques 10a, 10b de l'émetteur 3 peuvent présenter un angle inférieur à 180°, notamment un angle compris entre 140 et 170° entre leurs plans d'extension respectifs, par exemple un angle d'environ 160°.

L'émetteur 3 peut notamment comporter deux groupes de transducteurs 10a, 10b. Un premier groupe 10a peut comporter un ou plusieurs transducteurs utilisés en émission tandis que le deuxième groupe de transducteurs 10b peut comporter un ou plusieurs transducteurs utilisés en réception. Les fonctions des deux groupes de transducteurs 10a et 10b peuvent être échangées au cours du temps.

Sur les figures 5A à 5C, le premier et le deuxième groupe de transducteurs 10a, 10b comportent chacun un transducteur unique et sont symétriques.

Dans d'autres modes de réalisation, les deux groupes de transducteurs 10a, 10b peuvent être asymétriques et différents l'un de l'autre.

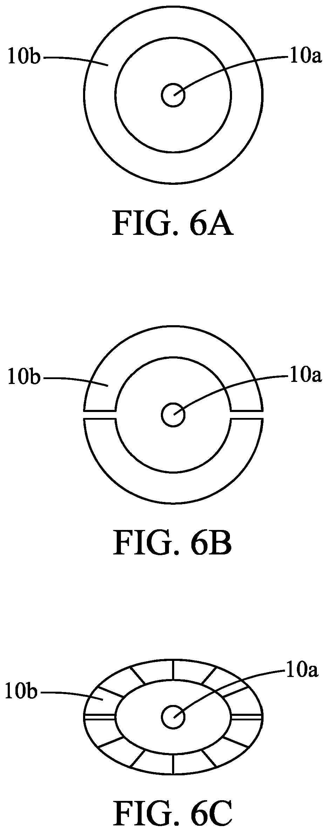

Par exemple sur la figure 6A, le premier groupe 10a comporte un unique transducteur 10a d'émission circulaire et central. Le deuxième groupe de transducteurs 10b comporte quant à lui un unique transducteur de réception 10b qui est en forme d'anneau autour du transducteur d'émission 10a.

Sur les figures 10B et 10C, le deuxième groupe de transducteurs comporte plusieurs transducteurs 10b (deux sur la figure 6B et 12 sur la figure 6C) qui sont disposés autour d'un transducteur d'émission centrale 10a de manière circulaire (sur la figure 6B) ou en forme d'ovale (sur la figure 6C).

Avantageusement, comme illustré sur la figure 4A, la face d'émission peut être terminée à deux extrémités transversales 7b, 7c opposées selon la direction transversale par deux portions de contact d'épaule 12, 9 sensiblement perpendiculaires à la face d'émission 7 et adaptées pour venir en appui contre un os de l'épaule de l'individu.

Ainsi en particulier le dispositif 1 peut être utilisé pour réaliser une mesure de flux sanguin à la fois sur la veine sous-clavière gauche et droite.

Dans ce mode de réalisation, le milieu de la zone active 11 est notamment situé sensiblement à équidistance des portions de contact d'épaule 9, 12 selon la direction transversale Y. Un écartement, selon la direction transversale Y entre les portions de contact d'épaule 9, 12 est ainsi sensiblement compris entre 40 et 100 millimètres, avantageusement compris entre 60 et 80 millimètres.

Pour permettre un positionnement encore plus précis, le boîtier 2 peut comprendre une face de calage 13.

La face de calage 13 peut être disposée sur le premier côté 5 du dispositif 1. La face de calage 13 est adaptée pour venir en appui plan contre la clavicule de l'individu.

Pour cela la face de calage 13 peut par exemple s'étendre sensiblement dans le plan d'extension verticale Y, Z et être disposée en retrait par rapport à la face d'émission 7 selon la direction longitudinale X de sorte à être adaptée pour venir en appui plan contre la clavicule de l'individu.

La face de calage 13 peut par exemple être disposée avec une distance de retrait par rapport à la face d'émission, selon la direction longitudinale X, comprise entre 5 et 15 millimètres.

Comme illustré sur les figures 4A et 4B, la face de calage 13 peut être reliée à la face d'émission 7 par l'intermédiaire de la portion de contact de clavicule 8.

En particulier, la portion de contact de clavicule 7 peut être reliée à la face de calage 13 par un congé 14.

Un tel congé 14 permet de suivre davantage la courbe de la clavicule de l'individu et donc d'améliorer la précision et la reproductibilité du positionnement du dispositif.

Le dispositif 1 est avantageusement compact et adapté pour pouvoir être aisément saisi d'une seule main par un plongeur souhaitant réaliser une mesure sur sa propre personne.

Pour cela le dispositif 1 peut notamment présenter un encombrement maximal dans le plan d'extension verticale Y, Z inférieur à 100 millimètres, avantageusement inférieur à 80 millimètres.

Comme illustré sur la figure 3, le boitier 2 accueille par ailleurs une électronique 4 de contrôle et de commande de l'émetteur acoustique.

L'électronique 4 est avantageusement disposée sur au moins une carte électronique 15 s'étendant sensiblement selon le plan d'extension verticale Y, Z.

Le boîtier 2 peut ainsi présenter un encombrement réduit selon la direction longitudinale X, par exemple un encombrement total selon la direction longitudinale X inférieur à 50 millimètres.

L'électronique 4 de contrôle et de commande de l'émetteur acoustique peut en particulier comporter des moyens de communication sans fil avec une station de base telle qu'un smartphone ou un ordinateur.

De cette manière, on simplifie encore le maniement du dispositif par un individu réalisant une mesure sur sa propre personne, en évitant la présence de câbles.

Par ailleurs, l'électronique 4 de contrôle et de commande de l'émetteur acoustique peut comporter une batterie rechargeable par induction.

On évite ainsi la présence d'une connectique et on renforce l'étanchéité et la tenue du dispositif dans des environnements hostiles comme la surface de l'eau par exemple.

## Revendications

1. Dispositif acoustique de mesure du flux sanguin dans une portion de système cardiovasculaire d'un individu pour des opérations de détection de bulles de décompression, le dispositif comprenant
un émetteur acoustique (3) comportant au moins un transducteur acoustique (10) adapté pour émettre un faisceau d'ultrasons et capter un signal ultrasonore réfléchi, et
un boitier (2) dans lequel est accueilli l'émetteur acoustique (3),
**caractérisé en ce que** le boitier (2) comprend une face d'émission (7), sensiblement plane ou convexe, venant coiffer l'émetteur acoustique (3) pour le protéger d'un environnement extérieur et assurer la transmission du faisceau d'ultrasons et du signal ultrasonore réfléchi,
la face d'émission (7) s'étendant sensiblement dans un plan d'extension verticale (Y, Z) comprenant une direction transversale (Y) et une direction verticale (Z) perpendiculaires de sorte à être apte à venir en contact avec la peau d'un individu selon une direction longitudinale (X) sensiblement perpendiculaire au plan d'extension verticale (Y, Z),
la face d'émission (7) étant terminée à une extrémité (7a) selon la direction verticale (Z) par une portion de contact de clavicule (8) sensiblement perpendiculaire à la face d'émission (7) et adaptée pour venir en appui contre un os de clavicule de l'individu,
la face d'émission (7) étant terminée à une extrémité (7b) selon la direction transversale (Y) par une portion de contact d'épaule (12) sensiblement perpendiculaire à la face d'émission (7) et adaptée pour venir en appui contre un os de l'épaule de l'individu,
une distance verticale (d2) entre un milieu de zone active (11) de la face d'émission (7) et la portion de contact de clavicule (8) étant inférieure à 20 millimètres, et
une distance transversale (d3) entre un milieu de zone active (11) de la face d'émission (7) et la portion de contact d'épaule (9) étant comprise entre 20 et 50 millimètres.

2. Dispositif acoustique selon la revendication 1, dans lequel la distance transversale (d3) entre le milieu de zone active (11) et la portion de contact d'épaule (9) est comprise entre 30 et 40 millimètres.

3. Dispositif acoustique selon l'une quelconque des revendications 1 et 2, dans lequel la face d'émission (7) est terminée à deux extrémités transversales (7b, 7c), opposées selon la direction transversale (Y), par deux portions de contact d'épaule (12, 9) sensiblement perpendiculaires à la face d'émission (7) et adaptées pour venir en appui contre un os de l'épaule de l'individu,
et dans laquelle le milieu de zone active (11) de la face d'émission (7) est situé sensiblement à équidistance des portions de contact d'épaule (9, 12) selon la direction transversale (Y).

4. Dispositif acoustique selon l'une quelconque des revendications 1 à 3, dans lequel le boîtier (2) comprend une face de calage (13) s'étendant sensiblement dans le plan d'extension verticale (Y, Z) et disposée en retrait par rapport à la face d'émission (7) selon la direction longitudinale (X) de sorte à être adaptée pour venir en appui plan contre la clavicule de l'individu, en particulier disposée avec une distance de retrait selon la direction longitudinale (X) comprise entre 5 et 15 millimètres.

5. Dispositif acoustique selon la revendication 4, dans lequel la portion de contact de clavicule (8) relie la face d'émission (7) à la face de calage (13), en particulier dans lequel la portion de contact de clavicule (8) est relié à la face de calage (13) en formant un congé (14) de sorte à suivre la courbe d'une clavicule de l'individu.

6. Dispositif acoustique selon l'une quelconque des revendications 1 à 5, comprenant en outre au moins un capteur de contact (15) de peau adapté pour détecter un contact de la face d'émission (7) avec la peau de l'individu, en particulier dans lequel le dispositif comporte deux capteurs de contact (15) disposés de part et d'autre de l'émetteur acoustique (3) selon la direction transversale (Y).

7. Dispositif acoustique selon l'une quelconque des revendications 1 à 6, dans lequel un transducteur (10) de l'émetteur acoustique (3) est incliné par rapport au plan d'extension verticale (Y, Z) de la face d'émission (7) de sorte à ce que la direction d'émission (X') du transducteur (10) soit inclinée en direction de la portion de contact de clavicule (8).

8. Dispositif acoustique selon l'une quelconque des revendications 1 à 7, dans lequel l'émetteur acoustique (3) présente un encombrement maximal dans le plan d'extension vertical (Y, Z) compris entre 30 et 40 millimètres.

9. Dispositif acoustique selon la revendication 8, dans lequel l'émetteur acoustique (3) comporte deux transducteurs acoustiques (10a, 10b).

10. Dispositif acoustique selon la revendication 8, dans lequel les deux transducteurs acoustiques présentent un angle compris entre 140 et 180° entre leurs plans d'extension respectifs.

11. Dispositif acoustique selon l'une quelconque des revendications 1 à 10, dans lequel le boîtier (2) accueille en outre une électronique (4) de contrôle et de commande de l'émetteur acoustique (3), ladite électronique étant disposée sur au moins une carte électronique s'étendant sensiblement selon le plan d'extension verticale (Y, Z).

12. Dispositif acoustique selon la revendication 11, dans lequel le boîtier (2) présente en outre un encombrement total selon la direction longitudinale (X) inférieure à 50 millimètres.

13. Dispositif acoustique selon la revendication 11 ou la revendication 12, dans lequel l'électronique (4) de contrôle et de commande de l'émetteur acoustique comporte des moyens de communication sans fil avec une station de base.

14. Dispositif acoustique selon la revendication 13, dans lequel la station de base est choisie parmi un smartphone et un ordinateur.

## Patentansprüche

1. Akustische Vorrichtung zum Messen des Blutflusses in einem Teil des Herz-Kreislauf-Systems einer Person für Vorgänge zur Detektion von Dekompressionsblasen, wobei die Vorrichtung umfasst:
einen akustischen Sender (3), der mindestens einen akustischen Wandler (10) umfasst, der dazu ausgelegt ist, einen Ultraschall-Strahl zu emittieren und ein reflektiertes Ultraschall-Signal aufzunehmen, und
ein Gehäuse (2), in dem der akustische Sender (3) untergebracht ist, **dadurch gekennzeichnet, dass** das Gehäuse (2) eine Emissionsfläche (7) aufweist, die im Wesentlichen plan oder konvex ist und den akustischen Sender (3) bedeckt, um ihn vor einer äußeren Umgebung zu schützen und die Übertragung des Ultraschall-Strahls und des reflektierten Ultraschall-Signals sicherzustellen,
wobei die Emissionsfläche (7) sich im Wesentlichen in einer vertikalen Ausdehnungsebene (Y, Z) erstreckt, die eine Querrichtung (Y) und eine vertikale Richtung (Z) umfasst, die zueinander senkrecht sind, um entlang einer Längsrichtung (X), die im Wesentlichen senkrecht zur vertikalen Ausdehnungsebene (Y, Z) verläuft, in Kontakt mit der Haut einer Person kommen zu können,
wobei die Emissionsfläche (7) an einem Ende (7a) in vertikaler Richtung (Z) durch einen Schlüsselbein-Kontaktabschnitt (8) abgeschlossen ist, der im Wesentlichen senkrecht zur Emissionsfläche (7) verläuft und dazu ausgebildet ist, in Anlage an einen Schlüsselbein-Knochen der Person zu kommen,
wobei die Emissionsfläche (7) an einem Ende (7b) in Querrichtung (Y) durch einen Schulter-Kontaktabschnitt (12) abgeschlossen ist, der im Wesentlichen senkrecht zur Emissionsfläche (7) verläuft und dazu ausgebildet ist, in Anlage an einen Knochen der Schulter der Person zu kommen,
wobei ein vertikaler Abstand (d2) zwischen einem Mittelpunkt des aktiven Bereichs (11) der Emissionsfläche (7) und dem Schlüsselbein-Kontaktabschnitt (8) kleiner als 20 Millimeter ist, und
ein Querabstand (d3) zwischen einem Mittelpunkt des aktiven Bereichs (11) der Emissionsfläche (7) und dem Schulter-Kontaktabschnitt (9) zwischen 20 und 50 Millimeter liegt.

2. Akustische Vorrichtung nach Anspruch 1, wobei der Querabstand (d3) zwischen dem Mittelpunkt des aktiven Bereichs (11) und dem Schulter-Kontaktabschnitt (9) zwischen 30 und 40 Millimeter liegt.

3. Akustische Vorrichtung nach einem der Ansprüche 1 und 2, wobei die Emissionsfläche (7) an zwei Querenden (7b, 7c), die in Querrichtung (Y) entgegengesetzt sind, durch zwei Schulter-Kontaktabschnitte (12, 9) abgeschlossen ist, die im Wesentlichen senkrecht zur Emissionsfläche (7) verlaufen und dazu angepasst sind, um an einem Knochen der Schulter der Person zur Anlage zu kommen,
und wobei der Mittelpunkt des aktiven Bereichs (11) der Emissionsfläche (7) in Querrichtung (Y) im Wesentlichen gleich weit von den Schulter-Kontaktabschnitten (9, 12) entfernt ist.

4. Akustische Vorrichtung nach einem der Ansprüche 1 bis 3, wobei das Gehäuse (2) eine Positionierungsfläche (13) aufweist, die sich im Wesentlichen in der vertikalen Ausdehnungsebene (Y, Z) erstreckt und relativ zur Emissionsfläche (7) in Längsrichtung (X) nach hinten zurückgesetzt ist, um angepasst zu sein, um flach in Anlage an das Schlüsselbein der Person zu kommen, insbesondere angeordnet mit einem Zurücksetzungsabstand in Längsrichtung (X) zwischen 5 und 15 Millimetern.

5. Akustische Vorrichtung nach Anspruch 4, wobei der Schlüsselbein-Kontaktabschnitt (8) die Emissionsfläche (7) mit der Positionierungsfläche (13) verbindet, insbesondere wobei der Schlüsselbein-Kontaktabschnitt (8) mit der Positionierungsfläche (13) verbunden ist, indem eine Ausrundung (14) gebildet wird, um der Krümmung eines Schlüsselbeins der Person zu folgen.

6. Akustische Vorrichtung nach einem der Ansprüche 1 bis 5, ferner umfassend mindestens einen Hautkontaktsensor (15), der zum Erfassen eines Kontakts der Emissionsfläche (7) mit der Haut der Person ausgebildet ist, wobei die Vorrichtung insbesondere zwei Kontaktsensoren (15) umfasst, die auf beiden Seiten des akustischen Senders (3) in Querrichtung (Y) angeordnet sind.

7. Akustische Vorrichtung nach einem der Ansprüche 1 bis 6, wobei ein Wandler (10) des akustischen Senders (3) relativ zur vertikalen Ausdehnungsebene (Y, Z) der Emissionsfläche (7) geneigt ist, so dass die Emissionsrichtung (X') des Wandlers (10) zum Schlüsselbein-Kontaktabschnitt (8) hin geneigt ist.

8. Akustische Vorrichtung nach einem der Ansprüche 1 bis 7, wobei der akustische Sender (3) in der vertikalen Ausdehnungsebene (Y, Z) einen maximalen Raumbedarf zwischen 30 und 40 Millimetern aufweist.

9. Akustische Vorrichtung nach Anspruch 8, wobei der akustische Sender (3) zwei akustische Wandler (10a, 10b) umfasst.

10. Akustische Vorrichtung nach Anspruch 8, wobei die beiden akustischen Wandler einen Winkel zwischen 140 und 180° zwischen ihren jeweiligen Ausdehnungsebenen aufweisen.

11. Akustische Vorrichtung nach einem der Ansprüche 1 bis 10, wobei das Gehäuse (2) weiter eine Elektronik (4) zur Steuerung und Bedienung des akustischen Senders (3) umfasst, wobei die Elektronik auf mindestens einer elektronischen Platine angeordnet ist, die sich im Wesentlichen entlang der vertikalen Ausdehnungsebene (Y, Z) erstreckt.

12. Akustische Vorrichtung nach Anspruch 11, wobei das Gehäuse (2) ferner einen Gesamtraumbedarf in Längsrichtung (X) von weniger als 50 Millimetern aufweist.

13. Akustische Vorrichtung nach Anspruch 11 oder Anspruch 12, wobei die Elektronik (4) zur Steuerung und Bedienung des akustischen Senders Mittel zur drahtlosen Kommunikation mit einer Basisstation umfasst.

14. Akustische Vorrichtung nach Anspruch 13, wobei die Basisstation aus einem Smartphone und einem Computer ausgewählt ist.

## Claims

1. An acoustic measurement device for the blood flow in a portion of the cardiovascular system of an individual, in particular for operations of decompression bubble detection, the device comprising:
an acoustic emitter (3) comprising at least one acoustic transducer (10) suited for emitting an ultrasound beam and detecting a reflected ultrasonic signal, and
a case (2) in which the acoustic emitter is received (3),
**characterized in that** the case (2) comprises an emission surface (7), substantially flat or convex, coming to cap the acoustic emitter (3) to protect it from an outside environment and to assure the transmission of the ultrasound beam and the reflected ultrasound signal,
the emission surface (7) extending substantially in a plane of vertical extension (Y, Z) comprising a transverse direction (Y) and a perpendicular vertical direction (Z) so as to be able to come into contact with the skin of an individual along a longitudinal direction (X) substantially perpendicular to the plane vertical extension (Y, Z),
the emission surface (7) being terminated at one end (7a) along the vertical direction (Z) by a clavicular contact portion (8) substantially perpendicular to the emission surface (7) and suited for coming to rest against a clavicle bone of the individual,
the emission surface (7) being terminated at one end (7b) along the transverse direction (Y) by a shoulder contact portion (12) substantially perpendicular to the emission surface (7) and suited for coming to rest against a bone of the shoulder of the individual,
a vertical distance (d2) between the middle of the active zone (11) of the emission surface (7) and the clavicular contact portion (8) being less than 20 mm, and
a transverse distance (d3) between the middle of the active zone (11) of the emission surface (7) and the shoulder contact portion (9) being included between 20 and 50 mm.

2. The acoustic device according to claim 1, wherein the transverse distance (d3) between the middle of the active zone (11) and the shoulder contact portion (9) is included between 30 and 40 mm.

3. The acoustic device according to any one of claims 1 and 2 wherein the emission surface (7) is terminated at two opposite transverse ends (7b, 7c) along the transverse direction (Y) by two shoulder contact portions (12, 9) substantially perpendicular to the emission surface (7) and suited for coming to rest against a bone of the shoulder of the individual,
and wherein the middle of the active zone (11) of the emission surface (7) is located substantially equidistant from the shoulder contact portions (9, 12) along the transverse direction (Y).

4. The acoustic device according to any one of claims 1 to 3 wherein the case (2) comprises an offset surface (13) extending substantially in the plane of vertical extension (Y, Z) and arranged recessed relative to the emission surface (7) along the longitudinal direction (X) so as to be suited for coming to press flat against the clavicle of the individual, in particular arranged with a recess distance along the longitudinal direction (X) included between 5 and 15 mm.

5. The acoustic device according to claim 4, wherein the clavicular contact portion (8) connects the emission surface (7) to the offset surface (13), in particular the clavicular contact portion (8) is connected to the offset surface (13) by forming a quarter hollow (14) so as to follow the curve of a clavicle of the individual.

6. The acoustic device according to any one of claims 1 to 5 further comprising at least one skin contact sensor (15) suited for detecting a contact of the emission surface (7) with the skin of the individual, in particular wherein the device comprises two contact sensors (15) arranged on either side of the acoustic emitter (3) along the transverse direction (Y).

7. The acoustic device according to any one of claims 1 to 6, wherein one transducer (10) of the acoustic emitter (3) is inclined relative to the plane of vertical extension (Y, Z) of the emission surface (7) such that the direction of emission (X') of the transducer (10) is inclined towards the clavicular contact portion (8).

8. The acoustic device according to any one of claims 1 to 7 wherein the acoustic emitter (3) has a maximum dimension in the plane of vertical extension (Y, Z) included between 30 and 40 mm.

9. The acoustic device according to claim 8, wherein the acoustic emitter (3) comprises two acoustic transducers (10a, 10b).

10. The acoustic device according to claim 8, wherein the two acoustic transducers have an angle between their respective planes of extension included between 140 and 180°.

11. The acoustic device according to any one of claims 1 to 10 wherein the case (2) further receives acoustic emitter (3) control and command electronics (4), where said electronics are laid out on at least one electronic card extending substantially along the plane of vertical extension (Y, Z).

12. The acoustic device according to claim 11, wherein the case (2) further has a total dimension along the longitudinal direction (X) of less than 50 mm.

13. The acoustic device according to claim 11 or claim 12, wherein the acoustic emitter control and command electronics (4) comprise means for wireless communication with a base station.

14. The acoustic device according to claim 13, wherein the base station is chosen among a smart phone and a computer.
